(19) Europäisches Patentamt / European Patent Office / Office européen des brevets

(11) **EP 2 736 439 B1**

(12) **EUROPEAN PATENT SPECIFICATION**

(45) Date of publication and mention
of the grant of the patent:
**21.12.2016 Bulletin 2016/51**

(21) Application number: **12819770.4**

(22) Date of filing: **27.07.2012**

(51) Int Cl.:
*A61B 18/22* (2006.01)     *A61N 5/06* (2006.01)
*A61N 5/067* (2006.01)     *A61B 18/00* (2006.01)
*A61B 17/00* (2006.01)     *A61B 90/98* (2016.01)

(86) International application number:
**PCT/US2012/048590**

(87) International publication number:
**WO 2013/019636 (07.02.2013 Gazette 2013/06)**

(54) **CLASS 1 LASER TREATMENT SYSTEM**

KLASSE-1-LASERBEHANDLUNGSSYSTEM

SYSTÈME DE TRAITEMENT LASER DE CLASSE 1

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2011 US 201161513213 P
25.07.2012 US 201213558021**

(43) Date of publication of application:
**04.06.2014 Bulletin 2014/23**

(73) Proprietor: **Biolitec Unternehmensbeteiligungs II
AG
1030 Vienna (AT)**

(72) Inventor: **NEUBERGER, Wolfgang
Dubai (AE)**

(74) Representative: **Herrmann, Franz
Dendorfer & Herrmann
Patentanwälte Partnerschaft mbB
Bayerstrasse 3
80335 München (DE)**

(56) References cited:
**EP-A1- 2 140 911     WO-A2-2008/033367
JP-A- 3 121 401     JP-A- 55 059 788
JP-A- 58 190 739     RU-A- 2004 133 704
US-A1- 2003 171 795     US-A1- 2008 212 623
US-A1- 2009 112 194**

• **"Safety of laser products - Part 1: Equipment
classification and requirements ;
60825_1_Ed_2p0_200", IEEE DRAFT;
60825_1_ED_2P0_2007-03, IEEE-SA,
PISCATAWAY, NJ USA, vol. 802.3, 1 February
2012 (2012-02-01), pages 1-202, XP017754422,
[retrieved on 2012-02-01]**

**Description**

**Background of the Invention**

**1. Field of the invention**

The present invention relates to minimally invasive medical treatment systems and in particular, eye-safe medical treatments using local energy emitting devices and conveying means.

**2. Prior Art Disclosure Statement**

Laser systems can operate as beneficial and effective medical instruments. They allow specific treatment to be administered with minimal invasiveness. Laser treatments are frequently preferred by those skilled in the art in different applications. There are an increasing number of medical applications involving the use of laser devices in various specialized disciplines such as angiology, proctoscopy, otolaryngology, urology, gynecology and aesthetics. Worth mentioning are treatment of insufficient veins, benign hyperplasic prostate (BPH), hemorrhoids, ulcers, fistulas, calculi, ear, nose and throat (ENT) disorders, and photodynamic treatments (PDT) just to name a few.

Laser energy devices must be designed and used with care in order to minimize the risk of accidents, especially those involving eye injuries. Even relatively small amounts of laser light can lead to permanent eye injuries. Thus, the manufacture, sale and usage of lasers systems are typically subject to government regulations.

Lasers are potentially hazardous because they can burn the retina of the eye, the cornea of the eye or even the skin. The low divergence angle of laser light along with the eye's focusing mechanism allow laser light to be concentrated in a very small area on the retina. Photoreceptor cells in the eye's retina can be damaged by an increase of just 10 degrees Celsius. With a powerful enough laser, permanent damage can occur almost instantly. The visible to near infrared laser radiation can penetrate the eyeball and cause heating of the retina, whereas exposure to laser radiation with wavelengths less than 400 nm and greater than 1400 nm are largely absorbed by the cornea and lens, which may cause development of cataracts or burn injuries. Infrared lasers with an emission wavelength in spectral region from 700 nm to 1400 nm are particularly hazardous, as the body's protective blink reflex response is not triggered by invisible light. Additionally, laser pulses shorter than about 1 μs can cause a rapid rise in temperature, resulting in explosive boiling of water. The resulting shock wave can subsequently cause damage relatively far away from the point of impact. Ultrashort pulses can also exhibit self-focusing in the transparent parts of the eye, leading to an increase of the damage potential in comparison to longer pulses with the same energy.

Currently used safety elements to prevent laser related accidents include wearing laser goggles, a door interlock system, a keylock system, a footswitch or a handswitch, an acoustical operation indicator, and an emergency shutoff button. Mentioned safety elements still present limitations and drawbacks or may be insufficient under some conditions.

Proper protective laser goggles have an effective optical density around a wavelength that is specific for the laser wavelength being emitted, which reduces transmission of dangerous levels of radiation. Therefore, whoever wears protective laser goggles designed for one specific wavelength range protects their eyes from laser radiation of that wavelength range. Today's standard laser goggles use an absorption process to prevent laser energy from entering the human eye. The goggles' lenses are made from 'colored' plastics in order to absorb incoming laser power. The resulting disturbed color impression is perceived by physicians as annoying. There are also laser protection goggles whose lenses are not made from colored plastics but have a dielectric coating that does not absorb but reflects incident laser radiation. The advantage of such goggles is an undisturbed color impression. However, the incident laser beam is reflected in an uncontrollable way and might endanger other persons. In some places it is not uncommon that, even when strongly recommended by laser manufacturer, not everyone in the treatment room wears safety goggles, especially, medical staff members who carry out tasks not directly involved with treatment and who mistakenly feel they are safe from laser irradiation. Finally, the costs of wavelength-specific goggles are significant and are not always easily acquired in many local markets. Furthermore, if more than one laser with different wavelengths or if a laser that emits at more than one wavelength is used in an operating room, safety goggles specific to each laser wavelength must be available and properly identified. Otherwise improper safety goggles may be selected and used mistakenly.

Door interlock systems are connected to the doors of a treatment room. Laser unit remains inoperative unless this interlock switch is closed. Therefore, if someone walks into the treatment room while laser is being emitted, the interlock system shuts off laser power, because the door interlock is not able to differentiate between persons who enter the treatment room with laser protection goggles on and those who do not wear them. Therefore, the door interlock system deactivates the laser emission every time someone enters the treatment room. As a consequence, the treatment could be unnecessarily interrupted at an uncontrollable, disadvantageous and even dangerous moment. The interruption/disturbance of a medical treatment might cause severe problems to the patient or could damage the success of the treatment. For such reasons, it is also not uncommon to see treatment rooms in which for different stated reasons, a door interlock system has not been installed and connected to room door.

Keylock systems do not allow radiation emission if enabling key is not inserted in place. Keys for keylock systems are often lost or misplaced and can easily be dented such that they no longer fit in lock, thus preventing treatments until replacement keys can obtained.

When a footswitch or hand switch is used, laser emits output radiation only as long as the user depresses the switch. However, foot switches or hand switches are often not desired because it is considered distracting for physicians to be required to maintain constant pressure on the switch throughout the procedure.

An acoustical operation indicator is usually an audible signal that is activated when laser is being emitted. Often such acoustic signals are annoying/distracting, especially when physicians carry out delicate and potentially dangerous treatments.

Emergency shutoff buttons are commonly large, red, easily accessible buttons that will instantly turn off power and shut down laser when pressed. Emergency buttons can easily be pressed by accident. Such interruptions are often designed to disallow immediate restarting the laser to make sure that 'problem' which caused the emergency shutdown is solved before restarting the laser device. At times lasers cannot be used or treatment is delayed because such emergency buttons are inadvertently in the pressed position.

For certain devices, such as lasers classified as Class 3B or Class 4 according to IEC standard 60601-2-22, a laser safety officer needs to be appointed at the hospital or operating site. Furthermore, in some countries such as in Germany, laser safety regulations require annual laser safety training for users of lasers with a laser class higher than class 1M as well as annual laser safety training for those employees, including nurses that work in an area where a laser of class 3B or 4 is operated. This represents an increase in treatment costs and requires careful and extensive management of human resources.

There are newer and more sophisticated safety mechanisms aimed at preventing the possibility of a laser fiber accidentally being pointed at or applied to someone in the treatment room while emitting laser energy and thus receiving stray or misdirected radiation in sensitive parts of the body such as the eyes. In vessel treatment applications, optical fiber is usually withdrawn from within diseased vessel as it is being irradiated, in order to cause obliteration along a diseased length. This pullback movement is generally carried out by means of some visual or sound-guided manual system or by means of an automatic motorized system. However, the danger of a fiber accidentally and inadvertently coming out of vessel while laser energy is being applied exists.

US patent 5,986,755 by Ornitz et al. discloses a safety device for detecting elastically scattered radiation comprising an excitation source of monochromatic radiation having a controllable output, a detector for detecting elastically scattered radiation collected from a specimen illuminated by the excitation source, and a signal conditioning circuit that comprises a transducer and a comparator. An output transducer signal representative of the elastically scattered radiation is compared with a predefined threshold signal. If the output transducer signal is less than the threshold signal, a control output signal coupled to the excitation source causes the output of the source to be reduced. The safety device is included with a Raman spectrometry apparatus. Thus device can be used to assess overtreatment of target tissue. US patent 7,413,567-B2 by Weckwerth et al. discloses a sensor for detecting the presence of skin, one configuration of which uses multiple light emitting diodes, each of a unique wavelength band, and a broad-band photodetector to measure the remission of light at multiple wavelengths from a material being analyzed. Characteristics of the spectral remission of the material are used to discriminate human skin from materials that are not human skin. Medical device utilizing such a sensor is inhibited from operation if skin has not been detected. These and other related inventions present limitations. For instance, invention by Ornitz et al. only controls the position of the fiber tip. Furthermore, Weckwerth's invention would be difficult to adapt to treatment of different types of human tissue. Therefore, a mechanism, that deactivates the laser when its beam hits human tissue, will not be helpful.

To control the risk of injury, various specifications, for example ANSI Z136 in the US and IEC 60825-1:2007 internationally, define "classes" of lasers according to their safety features depending on their power and wavelength. These regulations also prescribe required safety measures, such as exhibiting warning labels, including emergency shutoff systems and wearing laser safety goggles when operating lasers. Thus, Class 1 lasers are defined as safe under all conditions of normal use. This means the maximum permissible exposure (MPE) cannot be exceeded. The MPE is the highest power or energy density (in $W/cm^2$ or $J/cm^2$) of a light source that is considered safe, that is, that has a negligible probability for creating damage. The MPE is measured at the cornea of the human eye or at the skin, for a given wavelength and exposure time. A calculation of the MPE for ocular exposure takes into account the various ways light can act upon the eye. For example, deep-ultraviolet light causes accumulating damage, even at very low powers. Infrared light with a wavelength longer than about 1400 nm is absorbed by the transparent parts of the eye before it reaches the retina, which means that the MPE for these wavelengths is higher than for visible or ultraviolet light. In addition to the wavelength and exposure time, the MPE takes into account the spatial distribution of the light (from a laser or otherwise). Especially collimated laser beams of visible and near-infrared light can be dangerous at relatively low powers because the lens focuses the light onto a tiny spot on the retina. Light sources with a smaller degree of spatial coherence than a well-collimated laser beam, such as high-power LEDs, lead to a distribution of the light over a larger area on the retina.

Mentioned safety measures described in prior art are still not enough to be considered Class 1 devices. This is because a failure of any kind in such measures would not prevent a potential accident in which someone's eye is exposed to laser radiation.

The advantages of a laser Class 1 classification are that they are considered to be eye-safe under all conditions of normal use and therefore do not require safety systems and controls previously mentioned. Additionally, a laser emission control switch - usually a foot switch or hand switch - can be implemented as a simple switch. This switch may even be implemented, for example as a button on a touch screen or a button on a mouse-like device. Furthermore, many of mentioned treatments are done in outpatient / office. Thus, a Class 1 laser system would facilitate and allow cost reduction, which will reflect as a benefit to the patient.

Most of today's medical laser devices are classified as Class 3B or higher, as they can emit laser light at potentially hazardous energy levels. Thus, when medical laser treatments are carried out, laser goggles must be worn by everyone in the treatment room within the Nominal Ocular Hazard Distance (NOHD) and many of mentioned additional safety measures must also be applied.

Some Class 1 laser systems for medical applications exist in prior art:

In some PDT applications, a cylindrical diffuser lowers the emitted energy density to reach the requirements of laser Class 1 as long as they are used at levels required for initiating the phototoxic reaction for the PDT. Thus, the combination of a laser device with one or more cylindrical diffusers can be classified as a laser Class 1 system. However, a number of conditions must be met in order for these systems to be considered Class 1. First of all, the laser device must not operate with any other fiber probe other than with the cylindrical diffusers. Secondly the overall emitted laser power is limited in dependence on the length of the connected diffuser. Finally, the diffuser is built in a way that it cannot be broken in parts or that a break is immediately detected leading to a laser power reduction.

US Patent US 7,758,570-B2 by Walmsley discloses a device for low level laser therapy to induce a non-heating photochemical reaction. Device proposed claims to be a Class 1 laser device by including a laser generating means for generating a laser beam, the laser generating means having an apparent source size and homogenizing means for modifying the apparent source size of the laser beam. Such system is limited to treatment of conditions like tendonitis and other soft tissue injuries, wound healing and pain relief, leaving out a wide range of existing laser medical applications including treatment of diseased leg veins.

US Patent US 7,452,356-B2 by Groce et al. al presents a dermatologic treatment apparatus which includes a housing configured for manipulation in a dermatologic treatment procedure, a light source, and an electrical circuit. The circuit energizes the light source to produce output light pulses. A light path includes an aperture through which eye-safe light pulses are propagated having properties sufficient for providing efficacious treatment. An optical diffuser is disposed along the light path to reduce the integrated radiance to an eye-safe level. Once again, mentioned device is limited in that it is applicable to a restricted small range of dermatologic treatments.

According to previously mentioned prior art, there still remains a need for an eye-safe Class 1 laser system capable of performing a plurality of medical treatments. The present invention addresses these needs.

## Objectives and Brief Summary of the Invention

It is an objective of the present invention to provide a device for improved treatment of medical conditions.

It is another objective of the present invention to treat medical conditions accurately and safely, by using a localized, directed energy source and conveying means.

It is also an objective of the present invention to provide a device for safer, more reliable laser medical treatment by preventing accidental radiation from harming patients and medical staff.

It is yet another objective of the present invention to provide a laser system that is eye-sale for both patient and medical staff.

The invention is defined in the claims. Other embodiments are merely exemplary.

Briefly stated, an eye-safe, low-power-density, Class 1 or Class 3R laser treatment system for medical applications is disclosed. In a preferred embodiment, system has an output power and excess irradiation such that system meets requirements for laser safety according to IEC 60825-1:2007 or equivalent as eye-safe, and can be classified as laser Class 1 or as laser class 3R. Laser system comprises a diode laser source, an optical fiber probe, means for detecting and identifying said optical probe and means to ensure that laser power that is transmitted from the distal end of the fiber probe is limited to a pre-specified maximum power level according to application, laser wavelength, emission characteristics, probe characteristics and limiting values as defined by applying laser safety regulations. In another preferred embodiment, device additionally includes means to identify the connected optical fiber probe to prevent the use of non-conforming optical fiber probes and/or to automatically limit the maximum output power to an optical power that is in compliance with said laser safety regulations. System preferably operates at a wavelength of 1400 nm or higher. Additionally, device includes means to detect breakage or leak of optical fiber probe and to consequently shutdown laser emission if breakage or leak is detected.

The above and other objects, features and advantages of the present invention will become apparent from the following description read in conjunction with the accompanying drawings.

**Brief Description of Figures**

FIG. 1 depicts a preferred embodiment of present invention describing main components the system disclosed.
FIG. 2 depicts a bare fiber probe and main dimensions used for calculations of maximum emitted power.
FIG. 3 depicts a 360-degree circumferentially emitting probe whose main dimensions are used for calculations of maximum emitted power.

**Detailed Description of Preferred Embodiments**

The present invention addresses prior art disadvantages by assuring safe medical laser treatments with a system that prevents the possibility of accidental radiation from harming the patient or medical staff, particularly their eyes. This is achieved by means of a medical laser system design which complies with working condition requirements for laser safety to be considered as "Class 1" or as "Class 3R" or "eye safe" according to an official laser safety standard such as IEC 60825-1:2007, DIN EN 60825-1 or ANSI Z136.1.

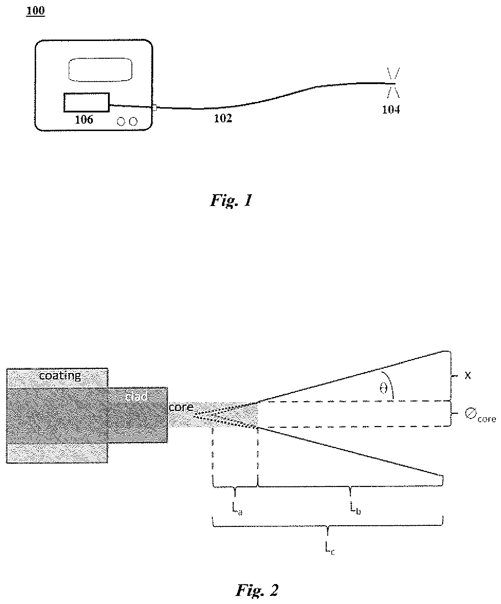

In a preferred embodiment, depicted in **FIG. 1**, laser system **100** comprises optical fiber probe **102** with firing end **104** which conveys energy from laser source **106,** preferably a diode laser source. Laser **106** is activated to emit electromagnetic radiation into target tissue directly in front of emitting face/(firing end) **104.** System comprises an intelligent safety control subsystem that can limit lasing parameters depending on desired safety and medical requirements. In a preferred embodiment, control system uses RFID technology to detect and recognize type of optical fiber probe **102** being used and limits lasing parameters according to pre-programmed standards to assure eye-safe conditions for everyone present. In another embodiment, control system detects breakage or leak of optical fiber probe and immediately shuts down laser emission. In another embodiment, user can manually input conditions required for medical application into control system **108** so control system can limit lasing parameters accordingly. In yet another preferred embodiment, control system detects if the distal end of optical fiber probe emits laser radiation outside the patient's body and thus reduces the laser output power to a safe level according to laser safety regulations or completely stops laser emission.

In a preferred embodiment, a 360° radial emitting fiber such as the one disclosed in US Patent Publication US 2009/0240242 by Neuberger et al., is used to carry out the procedure previously explained. Radial emission by this fiber is basically in a torus shape which is not only very efficient, but also minimizes power density applied in any given point in case of accidental radiation. In other preferred embodiments, optical fibers with different fiber tip configurations are employed. Variants include but are not limited bare fibers, cylindrical diffusers, circumferentially emitting fibers, off-axis fibers, and side firing fibers.

When laser radiation is applied to the human tissue, different wavelengths can be chosen. In a preferred embodiment, wavelength of approximately 1470 $\pm$ 60nm is used. However, other wavelengths above 1400 nm can be used with similar emission characteristics. In other embodiments, lower wavelengths, including but not limited to 980 nm and 1350 nm can be used, provided lasing parameters are set such that desired eye-safe emission or compliance with desired safety official standard classification is met. In another embodiment, laser system operates in pulsed mode with a pulse duration of 1 ms to 10 s.

The following example illustrates the basic calculations made for design of a preferred embodiment of present invention using a bare or a radial fiber to convey a wavelength of 1470 nm in continuous mode:

As shown in **Table 1** below, Standard IEC 60825-1:2007, summarizes three measuring conditions for a continuous wave radiation of a wavelength between 1400 and 4000 nm in order to determine device safety classification.

*Table 1*

| | Condition 1 | | Condition 2 | | Condition 2 | |
|---|---|---|---|---|---|---|
| | Applied to collimated beam where e.g. telescope or binoculars may increase the hazard | | Applied to diverging beam where e.g. magnifying glasses, microscopes may increase the hazard | | Applied to determine irradiation relevant for the unaided eye and for scanning beams | |
| Wavelength [nm] | Aperture stop [mm] | Distance [mm] | Aperture stop [mm] | Distance [mm] | Aperture stop [mm] | Distance [mm] |
| $\geq \geq 1400$ to 4000 | 7 x condition 3 | 2000 | 7 | 70 | 3.5 for $t \geq 10$ s | 100 |
| | | | | | | |

These conditions establish that power should be measured at a spherical surface of diameter $A_{meas, i}$ (where $i \in \{1, 2, 3\}$) at a distance of $L_{c, i}$ (where $i \in \{1, 2, 3\}$) from laser source. These conditions are:

Condition 1:

$L_{c, 1}$ = 2000mm

$A_{meas, 1}$ = 472 mm$^2$

Condition 2:

$L_{c,2}$ = 70mm
$A_{meas.2}$ = 39 mm$^2$

Condition 3:

$L_{c,3}$ = 100mm

$A_{meas\ 3}$ = 10 mm$^2$

According to standard IEC 60825-1:2007, for a device/system to be considered Class 1, the measured power MPP at these surfaces shall not be higher than:

$MPP_{Class1}$ = 10 mW.

The standard also determines that in order to not to harm the eye's cornea, the Maximum Permissible Exposure (MPE) of this tissue to laser energy shall be no greater than:

$I_{max}$(MPE) =1mW/mm$^2$.

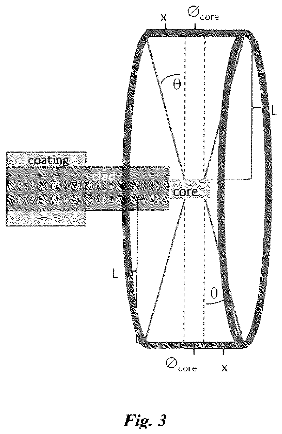

With these values in mind, estimations are presented for a bare fiber in Fig. 3 and for a radial fiber in **Fig. 4,** to learn the maximum power that can be emitted so that in each of the three mentioned conditions, the 10 mW limit is not exceeded:

Bare Fiber emitting face :

- $A_i = (x_i + r_{core})^2 \cdot \pi$ with $r_{core} = \varnothing_{core} / 2$ and index $i \in \{1, 2, 3\}$

- $\tan \theta = \dfrac{x_i}{L_{h,i}}$

$$NA = n_0 \cdot \sin\theta \quad \Rightarrow \quad \theta = \arcsin\left\{\frac{NA}{n_0}\right\} \text{ whereby } n_{0,\,air} = 1$$

$$x_i = L_{b,i} \cdot \tan\left\{\arcsin\left(\frac{NA}{n_0}\right)\right\} = L_{b,i} \cdot \tan\left\{\arcsin\left(\frac{0,26}{1}\right)\right\}$$

- $L_{b,j} = L_{c,j} - L_{a,i}$

Radial Fiber emitting face:

- $A_i = 2 \cdot \pi \cdot (L_i + r_{core}) \cdot (2x + 2r_{core})$ with $r_{core} = \varnothing_{core} / 2$ and index $i \in \{1, 2, 3\}$

- $\tan\theta = \dfrac{x_i}{L_i}$

$$NA = n_0 \cdot \sin\theta \quad \Rightarrow \quad \theta = \arcsin\left\{\frac{NA}{n_0}\right\} \text{ whereby } n_{0,\,Air} = 1$$

$$x_i = L_i \cdot \tan\left\{\arcsin\left(\frac{NA}{n_0}\right)\right\} = L_i \cdot \tan\left\{\arcsin\left(\frac{0,26}{1}\right)\right\}$$

Thus, to comply with IEC 60825-1:2007:

1) The maximum power $P_{class1}$ that a laser system can emit for a Class 1 laser system equivalence is the minimum value of $P_{class1,\,i}$ with $i \in \{1, 2, 3\}$ according to the three conditions mentioned above:

$$P_{class1,\,i} = 10mW \cdot A_i / A_{meas,\,i}$$

where $A_i$ is the area that is illuminated by the fiber probe at the given distance $L_{c,\,i}$.

2) The maximum power that a laser can emit to achieve an Eye-safe power density at a distance from the laser source (i.e. the fiber probe's end facet) to a person's eye with $L \geq 100mm$ is:

$$P_{max} = I_{max}\,(MPE) \cdot A$$

**Table 2** below summarizes the results of these estimations under each of the three mentioned conditions to comply with a Class 1 system rating for a wavelength range between 1400 and 4000 nm. Critical value shows the maximum value that complies with all three conditions, i.e. the lowest of the three. A homogeneous intensity distribution is assumed across the fiber core's cross section.

*Table 2: Class 1 values*

| Fiber | Critical value [W] |
|---|---|
| **Bare** | 40.5 |
| **Radial** | 6.0 |

**Table 3** below summarizes results of these estimations under each of the 3 conditions for a wavelength range between 1400 and 4000 nm to assure that an eye will not receive a higher power density than considered safe for the cornea (1 mW/mm$^2$). Calculations have been performed according to all three conditions and the most restrictive value was chosen, shown in the last column. In case of a "bare fiber" the critical value is 2.27 W, in case of a "RADIAL fiber" it is 23.39 W. Here again a homogeneous intensity distribution is assumed across the fiber core's cross section.

*Table 3 Eye-safe values*

| Fiber | Critical value [W] |
|-------|--------------------|
| **Bare** | 2.3 |
| **Radial** | 23.4 |

From the above calculations, the control system of the present invention's device can be programmed; for example, for a radial fiber conveying 1470 nm for varicose vein ablation such that energy is emitted in continuous mode at a maximum power of 6 W (see Table 2). Under this configuration, system is considered Class 1 and procedure can be carried out without needing to have door open shutoffs or to use safety goggles by everyone in the office. Furthermore, it can be seen from Table 3, that under normal working conditions, higher emission powers (up to 23 W), at the probe tip would still be considered eye-safe for a great amount of medical applications.

Similar calculations to these examples can be carried out to determine maximum values for a device to comply with Class 3R, that is:

$$MPP_{Class3R} = 50 \text{ mW}.$$

In another embodiment, control system can be programmed for pulsed mode with a pulse duration of 1 ms to 10 s and having a maximum output power of at least 3W.

In some embodiments, system additionally includes means to ensure that no laser power beyond critical value is emitted. In one embodiment, a safety system is incorporated which senses an interaction between ablation energy and aiming beam. Sensing system measures back-scattering from aiming beam and senses a difference on signal when fiber is outside or inside the body and limits or cuts lasing energy when fiber is outside the body according to how control system has been preprogrammed. In another embodiment, control system detects breakage of optical fiber probe and immediately shuts down laser emission. In yet another embodiment, user can manually input conditions required for medical application into control system **108** so control system can limit lasing parameters accordingly. In yet another embodiment, fiber is encapsulated within a metal tubing. This metal protective tubing prevents the optical fiber from being easily broken. Additionally it blocks radiation that could be emitted from a broken fiber.

The proper combination of wavelength and maximum radiation power applied, plus the fact that a 360° radial emission fiber is used, allows for a minimum power density of potentially stray radiation. As device has limited energy values for certain applications, low power allows for use of fibers with core diameter as small as 220 or 360 $\mu$m. The use of less expensive, smaller diameter fibers reduces the cost of procedure.

Present invention prevents injury to the unprotected eye, by applying radiation parameters such that power density of accidental radiation is insufficient to cause harm. Therefore, in endovenous treatments such as endovenous treatment of vein disorders and photodynamic therapy particularly benefit from the present invention, as safety is critical in these types of treatments. However a plurality of laser treatments can also benefit from present invention.

As a consequence of any or all of mentioned safety features, treatment reliability and safety are greatly enhanced. Additionally, outpatient / office treatments carried out with a Class 1 laser system would facilitate and allow cost reduction, which will reflect as a benefit to the patient.

Having described preferred embodiments of the invention with reference to the accompanying drawings, it is to be understood that the invention is not limited to the precise embodiments, and that various changes and modifications may be effected therein by skilled in the art without departing from the scope of the invention as defined in the appended claims.

**Claims**

1. A laser treatment system (100) for medical applications comprising:

a laser device (106);
an elongate optical transmission means, having a proximal end and a distal end (104);
first means for connecting said laser device reliably and controllably to said optical transmission means at its proximal end;

second means to ensure that laser power that is transmitted from the distal end of the optical transmission means is limited to a pre-specified maximum power level;

**characterized in that**

said maximum power level depends on laser wavelength, emission characteristics of said optical transmission means and limiting values so that said system in operation meets requirements for laser safety for designation as laser Class 1 or 1M or as laser class 3R system according to IEC 60825-1:2007 or equivalent; and that said system comprising further third means adapted to detect and identify said optical transmission means.

2. The system according to claim 1,
wherein said elongate optical transmission means is an optical fiber probe (102) and wherein the system further comprises means to detect breakage or leakage of said optical fiber probe and to shutdown laser emission upon breakage of said optical fiber probe.

3. The system according to claim 1 or 2,
wherein said laser device comprises a diode laser device or a laser-diode pumped solid-state laser.

4. The system according to claim 2 or 3,
wherein said third means adapted to identify said optical fiber probe (102) is a proprietary connector adapted to prevent the use of non-conforming optical fiber probes
or
said third means adapted to identify said optical fiber probe is based on RFID technology.

5. The system according to any one of claims 1 to 4,
wherein said laser device is adapted to operate at a wavelength higher than 1400nm and lower than 4000nm, preferably at a wavelength of 1470 ± 60 nm.

6. The system according to any one of claim 2 to 5,
wherein said optical fiber probes (102) are selected from the group consisting of bare fibers, cylindrical diffusers, circumferentially emitting fibers, off-axis fibers, and side firing fibers.

7. The system according to any one of claims 1 to 6,
wherein said maximum power level, radiated from the distal end of said optical transmission means is low enough to be harmless to a human eye according to IEC 60825-1:2007 or equivalent.

8. The system according to claim 7,
adapted to operate in continuous-wave mode and having a maximum output power in excess of 3W.

9. The system according to claim 7,
adapted to operate in pulsed mode with pulse duration of 1ms to 10s and having a maximum output power in excess of 3W.

10. The system according to any one of claims 1 to 9,
further comprising sensing and controlling means to detect if the distal end of said optical transmission means leaves a body cavity, a blood vessel or the inside of a tissue area such that laser radiation is accidentally emitted outside the patient's body to reduce the laser output power of the emitted laser radiation to a safe level according to laser safety regulations, and/or to completely shut down laser emission.

11. The system according to any one of claims 1 to 10,
wherein the system is arranged for endoluminal treatment of varicose veins.

12. The system according to any one of claims 1 to 10, wherein the system is arranged for photodynamic therapy.

**Patentansprüche**

1. Laserbehandlungssystem (100) für medizinische Anwendungen mit:

einem Lasergerät (106);

einem langgestreckten optischen Übertragungsmittel mit einem proximalen Ende und einem distalen Ende (104);

einem ersten Mittel, um das Lasergerät zuverlässig und überprüfbar mit dem optischen Übertragungsmittel an seinem proximalen Ende zu verbinden;

einem zweiten Mittel, um zu gewährleisten, dass die Laserleistung, die vom distalen Ende des optischen Übertragungsmittels übertragen wird, auf ein vorbestimmtes maximales Leistungsniveau begrenzt ist;

**dadurch gekennzeichnet, dass**

das maximale Leistungsniveau von der Laserwellenlänge, der Emissionscharakteristik des optischen Übertragungsmittels und begrenzenden Werten abhängt, so dass das System im Betrieb den Anforderungen an die Lasersicherheit für die Kennzeichnung als System nach Laserklasse 1 oder 1M oder als Laserklasse 3R gemäß IEC 60825-1:2007 oder einem Äquivalent genügt; und dass

das System weiter ein drittes Mittel aufweist, das dazu eingerichtet ist, das optische Übertragungsmittel zu erfassen und zu identifizieren.

2. System nach Anspruch 1,
bei dem das langgestreckte optische Übertragungsmittel eine optische Fasersonde (102) ist, wobei das System ferner ein Mittel umfasst, um ein Brechen oder eine Leckage der optischen Fasersonde zu erfassen und um die Laseremission beim Brechen der optischen Fasersonde abzuschalten.

3. System nach Anspruch 1 oder 2,
bei dem das Lasergerät ein Diodenlasergerät oder einen von einer Laserdiode gepumpten Festkörperlaser aufweist.

4. System nach Anspruch 2 oder 3,
bei dem das dritte Mittel, das dazu eingerichtet ist, die optische Fasersonde (102) zu identifizieren, eine proprietäre Steckverbindung ist, die dazu geeignet ist, die Verwendung von nicht übereinstimmenden optischen Fasersonden zu verhindern
oder
bei dem das dritte Mittel zum Identifizieren der optischen Fasersonden auf RFID-Technologie beruht.

5. System nach einem der Ansprüche 1 bis 4,
bei dem das Lasergerät für den Betrieb bei einer Wellenlänge größer 1400 nm und weniger 4000 nm, vorzugsweise bei einer Wellenlänge von 1470 ± 60 nm eingerichtet ist.

6. System nach einem der Ansprüche 2 bis 5,
bei dem die optischen Fasersonden (102) aus der Gruppe ausgewählt werden, die aus bloßen Fasern, zylindrischen Diffusoren, umfänglich emittierenden Fasern, außeraxialen Fasern und seitwärts abstrahlenden Fasern besteht.

7. System nach einem der Ansprüche 1 bis 6,
bei dem das maximale Leistungsniveau, das vom distalen Ende des optischen Übertragungsmittels abgestrahlt wird, niedrig genug ist, um für das menschliche Auge gemäß IEC 60825-1:2007 oder einem Äquivalent harmlos zu sein.

8. System nach Anspruch 7,
das für den Betrieb im kontinuierlichen Modus und mit einer maximalen Ausgangsleistung von mehr als 3 W eingerichtet ist.

9. System nach Anspruch 7,
das zum Betrieb im gepulsten Modus mit einer Pulsdauer von 1 ms bis 10 s und mit einer maximalen Ausgangsleistung von mehr als 3 W eingerichtet ist.

10. System nach einem der Ansprüche 1 bis 9,
das weiter ein Sensor- oder Überwachungsmittel aufweist, um zu erfassen, ob das distale Ende des optischen Übertragungsmittels einen Körperhohlraum, ein Blutgefäß oder das Innere eines Gewebebereichs verlässt, so dass Laserstrahlung zufällig außerhalb des Patientenkörpers emittiert wird, um die Laserausgangsleistung der emittierten Laserstrahlung auf ein sicheres Niveau gemäß den Lasersicherheitsvorschriften zu reduzieren und/oder die Laseremission komplett abzuschalten.

11. System nach einem der Ansprüche 1 bis 10,

bei dem das System für die endoluminale Behandlung von varikösen Venen eingerichtet ist.

**12.** System nach einem der Ansprüche 1 bis 10,
bei dem das System für die photodynamische Therapie eingerichtet ist.

**Revendications**

**1.** Un système de traitement au laser (100) destiné à des applications médicales comprenant :

un dispositif laser (106),
un moyen de transmission optique allongé possédant une extrémité proximale et une extrémité distale (104),
un premier moyen de raccordement dudit dispositif laser de manière fiable et commandable audit moyen de transmission optique au niveau de son extrémité proximale,
un deuxième moyen destiné à garantir qu'une puissance laser qui est transmise à partir de l'extrémité distale du moyen de transmission optique est limitée à un niveau de puissance maximal préspécifié, **caractérisé en ce que**
ledit niveau de puissance maximal dépend de la longueur d'onde du laser, de caractéristiques d'émission dudit moyen de transmission optique et de valeurs limites de sorte que ledit système en fonctionnement réponde à des exigences de sécurité laser pour une désignation en tant que système laser de classe 1 ou 1 M ou en tant que système laser de classe 3R conformément à IEC 60825-1:2007 ou équivalent, et que
ledit système comprenant en outre un troisième moyen adapté de façon à détecter et identifier ledit moyen de transmission optique

**2.** Le système selon la Revendication 1,
dans lequel ledit moyen de transmission optique allongé est une sonde à fibre optique (102), le système comprenant en outre un moyen de détection d'une rupture ou d'une fuite de ladite sonde à fibre optique et de suspension de l'émission laser en cas de rupture de ladite sonde à fibre optique.

**3.** Le système selon la Revendication 1 ou 2,
dans lequel ledit dispositif laser comprend un dispositif laser à diode ou un laser solide pompé par laser à diode.

**4.** Le système selon la Revendication 2 ou 3,
dans lequel ledit troisième moyen adapté de façon à identifier ladite sonde à fibre optique (102) est un connecteur propriétaire adapté de façon à empêcher l'utilisation de sondes à fibre optique non conformes ou
ledit troisième moyen adapté de façon à identifier ladite sonde à fibre optique est basé sur une technologie RFID.

**5.** Le système selon l'une quelconque des Revendications 1 à 4, dans lequel ledit dispositif laser est adapté de façon à fonctionner à une longueur d'onde supérieure à 1400 nm et inférieure à 4000 nm, de préférence à une longueur d'onde de 1470 ± 60 nm.

**6.** Le système selon l'une quelconque des Revendications 2 à 5, dans lequel lesdites sondes à fibre optique (102) sont sélectionnées dans le groupe se composant de fibres nues, de diffuseurs cylindriques, de fibres à émission circonférentielle, de fibres hors axe et de fibres à tir latéral.

**7.** Le système selon l'une quelconque des Revendications 1 à 6, dans lequel ledit niveau de puissance maximal rayonné à partir de l'extrémité distale dudit moyen de transmission optique est suffisamment faible pour être inoffensif pour un oeil humain conformément à IEC 60825-1:2007 ou équivalent.

**8.** Le système selon la Revendication 7,
adapté de façon à fonctionner en mode à onde continue et possédant une puissance de sortie maximale dépassant 3W.

**9.** Le système selon la Revendication 7,
adapté de façon à fonctionner en mode pulsé avec une durée d'impulsion de 1 ms à 10 s et possédant une puissance de sortie maximale dépassant 3W.

**10.** Le système selon l'une quelconque des Revendications 1 à 9, comprenant en outre un moyen de détection et de commande destiné à détecter si l'extrémité distale dudit moyen de transmission optique quitte une cavité corporelle, un vaisseau sanguin ou l'intérieur d'une zone tissulaire de sorte qu'un rayonnement laser soit émis accidentellement à l'extérieur du corps du patient de façon à réduire la puissance de sortie laser du rayonnement laser émis à un niveau sûr conformément à des réglementations de sécurité laser et/ou à suspendre totalement l'émission laser.

11. Le système selon l'une quelconque des Revendications 1 à 10, dans lequel le système est agencé pour un traitement endoluminal de veines variqueuses.

12. Le système selon l'une quelconque des Revendications 1 à 10, dans lequel le système est agencé pour une thérapie photodynamique.

*Fig. 1*

*Fig. 2*

*Fig. 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5986755 A, Ornitz
- US 7413567 B2, Weckwerth
- US 7758570 B2, Walmsley
- US 7452356 B2, Groce
- US 20090240242 A, Neuberger